# EUROPÄISCHE PATENTSCHRIFT

(11) **EP 1 578 526 B1**
(45) Veröffentlichungstag und Bekanntmachung des Hinweises auf die Patenterteilung: **18.05.2011**
(21) Anmeldenummer: 03808257.4
(22) Anmeldetag: 29.12.2003
(51) Int. Cl.: B01J 20/26, B01J 20/30, A61M 1/36, C08F 12/36

(54) **ADSORBERMATERIAL FÜR BLUT-, BLUTPLASMA- UND ALBUMINREINIGUNGSVERFAHREN**
ADSORBING MATERIAL FOR BLOOD AND PLASMA CLEANING METHOD AND FOR ALBUMIN PURIFICATION
MATERIAU ADSORBANT POUR PROCEDE D'EPURATION DU SANG ET DU PLASMA SANGUIN ET DE PURIFICATION DE L'ALBUMINE

(30) Priorität: 30.12.2002 DE 10261910
(43) Veröffentlichungstag der Anmeldung: 28.09.2005
(73) Patentinhaber: Polymerics GmbH, 12681 Berlin (DE)
(72) Erfinder: LEISTNER, Aniela, 12681 Berlin (DE); LEISTNER, André, 12681 Berlin (DE)
(74) Vertreter: Neumann, Günter
(86) Internationale Anmeldenummer: PCT/DE2003/004297
(87) Internationale Veröffentlichungsnummer: WO 2004/060554

(56) Entgegenhaltungen:
- EP-A- 0 319 144
- WO-A-02/059184
- DE-A- 19 922 268
- US-A- 4 202 775
- US-B1- 6 177 513
- US-B1- 6 423 024
- US-B2- 6 419 830

## Beschreibung

Die Erfindung betrifft ein Adsorbermaterial für Blut-, Blutplasma- und Albuminreinigungsverfahren sowie ein Verfahren zu seiner Herstellung.

Anwendungsgebiete für die Erfindung sind spezielle Blut- Blutplasma- und Albuminreinigungsverfahren in der Medizin und Pharmazie, die freie und albumingebundene Toxine sowie Giftstoffe aus dem Blut, Blutplasma bzw. Albumin entfernen. Diese Verfahren werden zur Behandlung von Arzneimittel- und Drogenvergiftungen, Vergiftungen mit Chemikalien und hochdosierten Chemotherapiemedikamenten, akutem und chronischem Nierenversagen, akuten und chronischen Lebererkrankungen (wie z.B. Hyperbilirubinämie, Cholestase, hepatische Encephalopathie, fulminantes Leberversagen) sowie Multiorganversagen eingesetzt.

Die konventionellen Methoden der Blutreinigung werden in Membrantechniken (Hämodialyse, Plasmapherese, Ultrafiltration), Adsorptionstechniken (Hämoperfusion, Plasmaperfusion) und in kombinierte Membran-Adsorptions-Techniken (z.B. MARS®-Verfahren) eingeteilt. Bei den Membrantechniken wird ein unerwünschter Stoff aus einem flüssigen Gemisch entfernt, indem man das Gemisch über eine semipermeable Membran mit einer Spüllösung in Kontakt bringt, die den zu entfernenden Stoff nicht enthält (Dialyse). Der Unterschied zwischen der Konzentration des zu trennenden Stoffes in dem Stoffgemisch und in der Spüllösung ist die Triebkraft für den Konzentrationsausgleich. Ist die Porengröße der semipermeablen Membran so, dass der zu trennende Stoff hindurchdringen kann, erfolgt der Konzentrationsausgleich durch Permeation des zu trennenden Stoffes in die Spüllösung. Bei der Hämolyse werden also Membranen eingesetzt, deren Poren groß genug sein müssen für die zu entfernenden Toxine und gleichzeitig klein genug, um die Blutbestandteile höherer Molekülgröße wie z.B. Albumin, Hämoglobin, Erythozyten, Leukozyten, Thrombozyten, nicht durchzulassen.

Bei den Adsorptionstechniken (Hämoperfusion, Plasmaperfusion) fließt das Vollblut bzw. nur das Blutplasma durch eine Säule bzw. Kartusche, die mit einem makroporösen Stoff wie Aktivkohle, Adsorberpolymer oder Ionenaustauscher gefüllt ist und wird dabei entgiftet. Bei der Plasmaperfusion müssen vor dem Adsorptionsschritt die Blutzellen zunächst abgetrennt und nach der Behandlung wieder mit dem Blutplasma zusammengeführt werden.

Zu den kombinierten Verfahren der extrakorporalen Blutreinigung gehört das MARS®-Verfahren (Molecular Adsorbent Recirculating System), das eine Kombination aus Dialyse und Perfusion darstellt. Bei dem MARS®-Verfahren wird das Blut des Patienten durch einen Albumindialysator geleitet und danach dem Patienten zurückgeführt. In dem Albumindialysator zirkuliert an der Waschseite reine Albuminlösung in einer Konzentration von 5-20%. Da die meisten Bluttoxine albumingebunden transportiert werden, entsteht so die Triebkraft für die Toxine, die Dialysemembran zu passieren. Die Albuminwaschlösung wird im Anschluß inline im Perfusionsblock gereinigt, danach einer Standarddialyse unterworfen und wieder zum Albumindialysator zurückgeführt. Mit diesem Prinzip gelingt es, die Entgiftungsfunktion der Leber zu ersetzen, welche bei Leberversagen primär lebensbedrohlich ist.

Hämodialyse, Ultrafiltration und Plasmapherese trennen die Bestandteile des Blutes nach ihrer Größe weitgehend unselektiv. Im Unterschied dazu können Sorptionstechniken sowohl sehr selektiv als auch weniger selektiv arbeiten. Die Membranverfahren benötigen maßgeschneiderte Membranen, die Sorptionstechniken benötigen maßgeschneiderte Adsorbermaterialien.

Als Adsorbermaterialien für extrakorporale Blutreinigungsverfahren werden neben der Aktivkohle zunehmend synthetische, makroporöse Adsorberpolymere eingesetzt. Triebkraft für diese Entwicklung sind die Nachteile von Aktivkohlematerialien. Hierzu zählen geringe mechanische Stabilität, geringe Selektivität und geringe Adsorptionsgeschwindigkeit, ferner hohe Rückhaltung von weißen Blutkörperchen und Blutblättchen und die Initiierung von Blutgerinnseln.

In der Literatur und in Patenten werden folgende polymere Adsorbermaterialien beschrieben:
- poröse und hochporöse Styren-Divinylbenzen-Copolymere,
- makroporöse Divinylbenzen-Copolymere,
- makroporöse Methacrylat- bzw.- Acrylat-Co- und Terpolymere,
- poröse, perlförmige Cellulosederivate.

Adsorbentien aus Aktivkohle bzw. aus beschichteter Aktivkohle, beispielsweise mit einer Lösung aus Polyacrylsäure oder aus Polyacrylsäure und Polyethylenimin wurden in SU 732 207 beschrieben. Weitere polymere Beschichtungen von Aktivkohle wurden in US 4,048,064, US 4,171,283, US 5,240,601, SU 844 769 beansprucht. Die Nachteile von Aktivkohlen wie die geringe mechanische Stabilität und geringe Adsorptionsgeschwindigkeit konnten dadurch jedoch nicht beseitigt werden.

Makroporöse Styren-Divinylbenzen-Copolymere wurden zur Entfernung von Barbituraten und Glucothimiden aus dem Hundeblut 1974 in US 3 794 584 eingesetzt. In der Folgezeit zeigte sich aber, daß sie in hohem Maße unpolar, unselektiv und blutunkompatibel sind. Hochporöse Styren-Divinylbenzen-Copolymere mit spezifischer Oberfläche über 800 m²/g, erhalten durch Nachvernetzung von schwachvernetzten Styren-Divinylbenzen-Copolymeren mit Hilfe von Monochlordimethylether in Gegenwart von Friedel-Crafts-Katalysatoren, wurden zwölf Jahre später 1986 ebenfalls als besonders effektive und schnelladsorbierende Materialien für die Hämoperfusion in DD 249 274 A1 beschrieben. Auch diese Adsorberpolymere haben unzureichende Blutkompatibilität und erfordern zusätzliche Nachbehandlungen. Ferner ist ihre Herstellung durch den Einsatz des cancerogenen Monochlordimethylether mit hohen Risiken für die Menschen und die Umwelt verbunden.

Durch spezielle und aufwendige Modifizierung der Oberfläche von hochporösen Styren-Divinylbenzen-Copolymeren mit Trifluoralkoxyphosphazenen (US 5,773,384) wird die Blutkompatibilität verbessert. Die nachträgliche Modifizierung verringert jedoch die spezifische Oberfläche und Porenzugänglichkeit und steigert enorm den Reinigungsaufwand, denn alle überschüssigen Reagenzien und Katalysatoren müssen aus den Mikro-, Meso- und Makroporen des Polymeren vor dem Einsatz in der Hämoperfusion entfernt werden.

Die Modifizierung der Oberfläche von hochvernetzten Divinylbenzen-Copolymeren durch Beschichtung oder Pfropfpolymerisation ist Gegenstand der vor kurzem angemeldeten Patente US 6,419,830 (2001) und US 6,423,024 (2002) zur Herstellung von hochporösen, kugelförmigen Divinylbenzenharzen für die Absorption von gesundheitsschädlichen Stoffen wie z.B. β-2-Microglobulin aus Blut oder Plasma. Für die Beschichtung werden kommerziell erhältliche, hochporöse Divinylbenzen-Copolymere eingesetzt, die aus 60 bis 90% Divinylbenzen bestehen, eine spezifische Oberfläche von 200 bis 1600 m²/g aufweisen, Porengrößen von 20 bis 500 Å mit einem Porengesamtvolumen bis 2,5 ml/g haben und im Korngrößenbereich von 25 bis 2500 µm erhältlich sind. Die hämokompatiblen Schichten werden durch Reaktion der restlichen Vinylgruppen auf der Oberfläche der DVB-Copolymeren mit hämokompatiblen Monomeren oder Polymeren erhalten, bestehend aus Phosphatidylcholin, Heparin, Polyalkylenglykolen, Polyalkoxyphosphazenen und Polyvinylpyrrolidon. Weitere beanspruchte Beschichtungen bestehen aus verschiedenen Vinylpyridin-, Vinylimidazol- und Vinylpyrrolidon-Derivaten sowie verschiedenen Acrylsäure- und Methacrylsäurederivaten. Der offensichtliche Nachteil dieser beiden Patente ist, daß die hämokompatible Beschichtung nachträglich erfolgt und wie schon oben aufgeführt ebenfalls zur Reduktion der spezifischen Oberfläche und Porenzugänglichkeit führt. Ein weiterer Nachteil dieses Verfahrens ist die vollständige Entfernung der zur Beschichtung verwendeten Monomeren, die in ihrer monomeren Form oft stark gesundheitsschädigend oder sogar cancerogen sind. Die Reinigung der auf diese Weise hämokompatibel modifizierten Polymere ist also aufwendig, kostspielig und nicht ganz risikofrei.

Die aus Polymerlösungen aufgebrachten hämokompatiblen Beschichtungen haben dagegen oft den Nachteil, dass sie sich in wässriger isotonischer Kochsalzlösung bzw. in Körperflüssigkeiten teilweise lösen und so unkontrolliert in den Blutkreislauf des Patienten gelangen können.

Das US 6,177,513 beschreibt eine Template-Polymerisation in dünnen Schichten unter Verwendung eines funktionellen Monomeren, eines Vernetzers, unter anderem von Divinylbenzen, einer Templateverbindung und gegebenenfalls eines Lösungsmittels. Die Polymerisation wird in der Regel als Lösungspolymerisation unter Stickstoffatmosphäre ausgeführt und liefert dünne Polymerfilme auf der Innenseite des Polymerisationsgefäßes, aus denen die Template-Verbindung mit Hilfe eines geeigneten Lösungsmittels herausgewaschen wird. Die erhaltenen Template-Filme eignen sich zwar zur hochselektiven Adsorption von organischen Substanzen im analytischen Maßstab, zur Blutreinigung sind jedoch die unter Stickstoffatmosphäre durch radikalische Suspernsionspolymerisation von Divinylbenzen und Vinylimidazol hergestellten Polymerisate nicht geeignet.

Aus der DE 199 22 268 A geht ein Verfahren zur Herstellung von perlförmigen, hochporösen, Adsorberpolymeren hervor, die durch radikalische Polymerisation eines Monomerengemisches aus Divinylbenzen, Ethylvinylbenzen und anderen Monomeren in einer dispersions- oder Suspensionspolymerisation in Gegenwart eines Initiators und einer inerten organischen Substanz oder eines Gemisches solcher Substanzen mit definierten Löslichkeitsparametern hergestellt werden. Mit diesem Verfahren verbindet sich jedoch der Nachteil, dass es nicht mit hydrophilen Monomeren, wie Vinylimidazol, durchgeführt werden kann.

Die US-A-4,202,775 offenbart ein Verfahren zur Abtrennung von organischen Verbindungen von Plasmaproteinen des Blutes, an denen die organischen Verbindungen adsorbiert sind, bei dem ein poröses Copolymerisat eingesetzt wird, das durch Copolymerisation eines Monomergemisches aus einem vernetzbaren Monomeren und einem monoethylenisch ungesättigten Monomeren hergestellt wird und das Poren mit einem mittleren Durchmesser (d) im Bereich von 500 Å bis 6000 Å aufweist, wobei das Volumen der Poren mit einem mittleren Durchmesser im Bereich von 0,5 d bis 2 d nicht mehr als 60 % des gesamten Porenvolumens des Copolymerisates beträgt.

Sofern dieses Adsorbermaterial zur Blutreinigung eingesetzt werden soll, ist eine Beschichtung seiner Oberfläche mit Plasmaproteinen erforderlich, die anschließend vernetzt werden müssen.

Als Problem erweist sich bei diesem Adsorbermaterial die auf Grund des großen mittleren Porendurchmessers verhältnismäßig geringe Kapazität, Adsorptionsgeschwindigkeit und mechanische Stabilität.

Aufgabe der vorliegenden Erfindung ist es daher, ein verbessertes poröses, vorwiegend kugelförmiges Adsorbermaterial zur Verfügung zu stellen, das eine höhere Adsorbtionskapazität, Adsorbtionsgeschwindigkeit und mechanische Stabilität aufweist und das vor der Verwendung für Blut-, Blutplasma- und Albuminreinigungsverfahren keiner Oberflächenbeschichtung bedarf.

Ferner soll ein Verfahren bereitgestellt werden, mit dem ein derartiges Adsorbermaterial einfach und mit vertretbarem ökonomischem Aufwand herstellbar ist.

Erfindungsgemäß wird die Aufgabe durch ein Adsorbermaterial für Blut-, Blutplasma- und Albuminreinigungsverfahren auf der Basis vernetzter, poröser Imidazol-Divinylbenzen-Copolymere gelöst, das durch radikalische Suspensionspolymerisation eines Monomergemisches aus Divinylbenzen-Vernetzer und einem Imidazol-Derivat hergestellt ist, wobei die Polymerisation in Gegenwart von Luft und/oder Sauerstoff, eines Salzes, eines Stabilisators und eines Inertstoffes erfolgt, das Imidazol-Derivat mit einem Anteil von 4 Gew. % bis 30 Gew. % im Adsorbermaterial enthalten ist, das Adsorbermaterial eine spezifische Oberfläche von 200 m²/g bis 900 m²/g und einen mittleren Porendurchmesser von 100 bis 500 Å sowie ein Gesamtporenvolumen von 1,0 cm³/g bis 2,0 cm³/g aufweist, wobei in 1 g des Materials bis 0,3 cm³ Mikroporen, bis 1,2 cm³ Mesoporen und bis 0,5 cm³ Makroporen enthalten sind, das Adsorberpolymer vorwiegend kugelförmig in einem Korngrößenbereich von 1µm bis 300 µm, vorzugsweise 50µm bis 200 µm oder 1µm bis 50 µm ausgebildet ist.

Vorteilhafte Ausgestaltungen des erfindungsgemäßen Adsorbermaterials ergeben sich aus den Merkmalen der Ansprüche 2 bis 6.

Gefunden wurde des Weiteren die Anwendung des erfindungsgemäßen Adsorbermaterials für das Adsorbieren im Molecular Adsorbent Recirculating System (MARS®-Verfahren).

Erfindungsgemäß wird die Aufgabe des Weiteren durch ein Verfahren gemäß den Merkmalen des Anspruches 7 zur Herstellung des Adsorbermaterials mit den Merkmalen der Ansprüche 1 bis 6 gelöst. Vorteilhafte Ausführungen des Verfahrens ergeben sich aus den Merkmalen der Ansprüche 8 und 9.

Das erfindungsgemäß hergestellte Adsorbermaterial weist im Vergleich zu der herkömmlichen Aktivkohlen und anderen kommerziellen Adsorbermaterialien wesentlich höhere Adsorptionskapazitäten und Adsorptionsgeschwindigkeiten gegenüber von freien und albumingebundenen Toxinen sowie Giftstoffen auf, insbesondere gegenüber von Bilirubin-Albumin-Komplexen (B-HSA-Lösungen) und gegenüber von freien Gallensäuren.

Ferner adsorbiert das erfindungsgemäße Adsorbermaterial ebenfalls N-Acetyltryptophan, Octansäure, Fettsäuren, Phenole und Coffein mit wesentlich höherer Geschwindigkeit und Kapazität als die kommerziell erhältlichen Adsorbermaterialien. Außerdem besitzt dieses Adsorbermaterial gute Biokompatibilität, wie in Cytotoxizitätstests und Hämolysetests gezeigt werden konnte. Das Adsorbermaterial lässt sich ohne Beeinträchtigung seiner vorteilhaften Eigenschaften sterilisieren und aufgrund seiner guten mechanischen Stabilität abriebfrei behandeln. Bei der Anwendung in Säulen und Kartuschen zeigt es vorteilhaftes Strömungsverhalten, so dass Durchflussgeschwindigkeiten bis 200 ml/min realisierbar sind. Das erfindungsgemäße Adsorbermaterial wurde in Bilirubin-Humanserum-Albumin-Lösungen sowohl im Batch-Verfahren (statischer Bilirubintest) als auch unter Kreislaufbedingungen (dynamischer Bilirubintest) geprüft.

Das erfindungsgemäße Adsorbermaterial wird nach dem Verfahren der Suspensionspolymerisation in Gegenwart von ausgewählten inerten Stoffen, Suspensionsstabilisatoren und Luft und/oder Sauerstoff hergestellt. Die Inertstoffe werden nach der Polymerisation wieder aus dem Polymeren entfernt. Sie sind in Verbindung mit dem Anteil an DVB im Polymerisationsansatz für den Grad der Porosität verantwortlich, d.h. für die Porengrößenverteilung und Porenvolumen.

Die Definition der Poren erfolgte nach IUPAC. Danach werden Mikroporen definiert als Poren mit einem Porendurchmesser unter 20 Å, Mesoporen sind Poren zwischen 20 Å und 500 Å und Makroporen sind Poren größer als 500 Å.

Als verwendbare Inertmittel seien beispielsweise genannt aliphatische und aromatische Kohlenwasserstoffe, höhermolekulare Alkohole, Ester, bzw. geeignete Polymerlösungen. Bevorzugte erfindungsgemäße Inertstoffe sind: Toluen, Dichlorethan, Tetrachlorkohlenstoff, Butylacetat, Ethylacetat, einzeln oder als Gemisch. Der Anteil des Inertstoffes in der organischen Phase kann im Bereich von 25 bis 50 Ma% variiert werden.

Die Aufgabe der Suspensionsstabilisatoren ist, die Koagulation der Tröpfchen während der Polymerisation zu verhindern. Geeignete erfindungsgemäße Suspensionsstabilisatoren sind wasserlösliche synthetische und natürliche Polymere, z.B. Polyvinylalkohol, teilweise verseiftes Polyvinylacetat, Methylcellulose, Hydroxyethylcellulose, Polyacrylsäure-Natriumsalze, Natriumsalz der Carboxymethylcellulose, ferner Pickering-Stabilisatoren, beispielsweise Calciumphosphat, Bentonite, Montmorillonite, Aluminiumhydroxid, Magnesiumhydroxid, Calciumcarbonat.

Die Suspensionspolymerisation wird mit Hilfe von monomerlöslichen radikalischen Initiatoren gestartet. Geeignete erfindungsgemäße Initiatoren sind Dibenzoylperoxid, Methylethylketonperoxid, Azoisobutyronitril. Vorzugsweise wird die Initiatormenge von 0,2 bis 2,0 Ma% bezogen auf das Gewicht des Monomergemisches verwendet.

Die Erfindung wird im Folgenden anhand nachstehender Beispiele näher erläutert.

### Beispiel 1

### Herstellung eines erfindungsgemäßen Adsorbermaterials (AM1)

In einem ummantelten 250-ml-Zylindergefäß, ausgestattet mit KPG-Rührer, Rückflußkühler, Temperaturfühler und 2 Schikanen werden 0,28 g Polyvinylalkohol mit einer Molmasse von 49000 g/mol in 142,5 g deionisiertem Wasser bei 30 °C gelöst. Danach werden 7,5 g NaCl zugegeben und die Lösung auf 70 °C gebracht. Die Rührgeschwindigkeit wird dabei auf 650 U/min eingestellt. Die Monomermischung, bestehend aus 18,7 g Divinylbenzen, 12,5 g 1-Vinylimidazol, 18,7 g Ethylacetat und 0,25 g AIBN, wird auf einmal zugegeben. Die entstandene Emulsion wird bis zur Ausbildung stabiler Tröpfchengröße 60 min bei 70 °C gerührt, danach auf 80 °C erhitzt und bei dieser Temperatur 10 h polymerisiert. Die entstandene Suspension wurde danach auf Raumtemperatur abgekühlt, auf einer Filternutsche abfiltriert, zunächst mit 3fachem Bettvolumen an deionisiertem Wasser und danach mit 2fachem Bettvolumen Ethanol gewaschen. Anschließend wurde der Filterkuchen 12 h bei 100 °C in einem Vakuumtrockenschrank getrocknet.

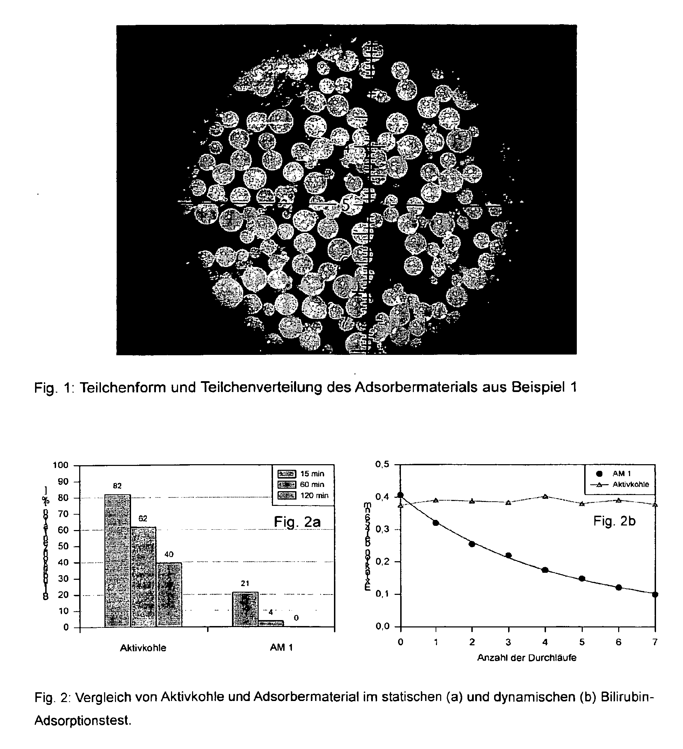

Die Zusammensetzung des Copolymers wurde mit Hilfe der Elementaranalyse bestimmt. Der gefundene Stickstoffwert betrug 6,2%, das entspricht 20,8 Ma% bzw. 26,7 Mol% an Vinylimidazol in dem Divinylbenzen-Vinylimidazol-Copolymeren. Die Ausbeute betrug 22 g. Die erhaltenen Partikel waren kugelförmig und lagen im Korngrößenbereich von 50 bis 150 µm (Fig. 1). Die spezifische Oberfläche, bestimmt mit der BET-Stickstoffadsorption, betrug 509 m²/g. Das Gesamtporenvolumen lag bei 1,3 ml/g und setzte sich zusammen aus 0,25 ml/g Mikroporen, 0,75 ml/g Mesoporen und 0,3 ml/g Makroporen in 1 g des Copolymeren.

### Beispiel 2

### Bestimmung des Adsorptionsverhaltens gegenüber von Bilirubin

Die Eignung der erfindungsgemäßen Adsorbermaterialien für die extrakorporale Blutreinigung wurde mit Hilfe eines statischen und eines dynamischen Bilirubin-Adsorptionstests bewertet und mit den zur Zeit in der Medizin üblichen Aktivkohleadsorbenzien verglichen. Es ist bekannt, dass das menschliche Albumin das Bilirubin besonders stark bindet. Aufgrund dessen wird das Adsorptionsverhalten verschiedener Adsorbentien gegenüber Bilirubin-Humanserum-Albumin-Komplexen stellvertretend für zahlreiche weitere albumingebundene Bluttoxine und Giftstoffe betrachtet.

Bilirubin ist ein Abbauprodukt des Hämoglobins (roter Blutfarbstoff) und kommt in den Gallenfarbstoffen vor. Bei Gelbsuchtkranken tritt es in erhöhtem Maße im Blut auf und verursacht die charakteristische Gelbfärbung der Patienten. Bilirubin entsteht normalerweise in der Milz durch oxidative Spaltung des Porphyrin-Rings des Häms und nachfolgende Hydrierung des grünen Zwischenprodukts Biliverdin. Als Entkoppler oxidativer Phosphorylierung ist Bilirubin hochgiftig für den Organismus und wird deshalb in der Blutbahn an Serum-Albumin gebunden und zur Leber transportiert. Bei Patienten mit akutem Leberversagen kann die Leber das Albumin nicht vom Bilirubin befreien. Diese Aufgabe der Leber sollen selektive Adsorberharze in den Perfusionsverfahren und gegebenenfalls eine selektive Membran in der MARS®-Anlage übernehmen.

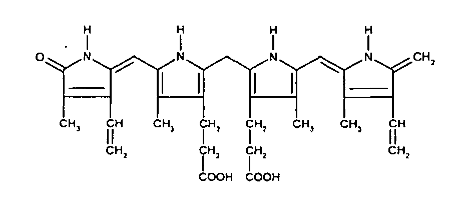

### Struktur von Bilirubin

### Herstellung der Testlösung

In Analogie zu den Verhältnissen im menschlichen Organismus wird das Bilirubin zunächst mit Humanserum-Albumin komplexiert und dieser Komplex als B-HSA-Lösung bezeichnet. Hierzu werden 33 mg Bilirubin in ein Eppendorfgefäß eingewogen, mit 1,25 ml 0,1 m NaOH versetzt und unter Lichtausschluß innerhalb von 5 min im Ultraschallbad gelöst. Nach der Auflösung wird der gesamte Inhalt des Eppendorfgefäßes quantitativ in eine braune 100-ml-Flasche überführt, in der vorher 28 ml 20%ige HSA-Lösung (Aventis) und 84 ml 0,9%ige NaCl-Lösung vorgelegt wurden. Die so hergestellte B-HSA-Lösung enthält 5 Ma% Albumin und 504 µmol Bilirubin pro Liter B-HSA-Lösung.

### Konditionierung der Adsorbermaterialien

500 mg des getrockneten Adsorbermaterials werden in ein SPE-Säulchen eingewogen und auf einer SPE-Vakuumstation plaziert. Das Adsorbermaterial wird jetzt nacheinander 2 mal mit 5 ml 70 %iger Ethanollösung versetzt, 5 mal mit 5 ml Wasser gewaschen und anschließend 3 mal mit 0,9 %iger NaCl-Lösung gewaschen und dann 2 min trockengesaugt.

### Adsorptionsversuch (Batch-Verfahren)

500 mg des konditionierten Adsorbermaterials werden in einem Schüttelgefäß mit 5 ml B-HSA-Lösung versetzt. Anschließend werden die Proben in einem Laborschüttler intensiv geschüttelt. In Zeitabständen von 15, 60 und 120 min wird der Schüttelversuch unterbrochen und aus der überstehenden Lösung 100 µl Probe für die UV-spektroskopische Bestimmung der Bilirubin-Restkonzentration entnommen, mit 0,9 ml 0,9 %iger NaCl-Lösung verdünnt und in einer 0,2-cm-Küvette vermessen.

Für die Bestimmung der Bilirubin-Restkonzentration wird das langwellige Absorptionsmaximum bei 453 nm genutzt. Die Änderung dex Extinktionsmaximums dieser Bande entspricht der Änderung der Bilirubinkonzentration in der B-HSA-Lösung. Als Referenzwert dient die Extinktion der B-HSA-Lösung vor dem Kontakt mit dem Adsorbermaterial. So lassen sich relative Konzentrationsänderungen leicht ermitteln. Üblicherweise wird die verbleibende Bilirubinkonzentration nach 15, 60 und 120 min als Gruppe von 3 Zahlenwerten in % angegeben. Die Referenz-B-HSA-Lösung enthält 5% Humanserum-Albumin und 505 µmol/l Bilirubin. Die Ergebnisse zeigt Fig. 2a.

### Dynamischer Bilirubintest (Kreislaufverfahren)

Zum Vergleich des erfindungsgemäßen Adsorbermaterials mit der zur Zeit in der Medizin üblicherweise eingesetzten Aktivkohle wurde ein dynamischer Bilirubin-Adsorptionstest entwickelt. Dieser Test versucht, die Bedingungen in einem konventionellen Perfusionsverfahren bzw. in einer MARS®-Anlage zu simulieren. Hierzu wird das Adsorbermaterial in Festphasenextraktionssäulen ("Minikartuschen") gegeben und auf eine SPE-Station aufgesetzt. Es wird dann wie oben konditioniert und anschließend mit B-HSA-Lösung versetzt. Mit Hilfe leichten Unterdrucks wird die Lösung durch das Adsorbermaterial durchgesaugt und im Filtrat die Bilirubinkonzentration UV-spektroskopisch bestimmt. Das Filtrat wird anschließend erneut in die Säule gegeben und die gesamte Prozedur 7 mal wiederholt. Die Ergebnisse zeigt Fig. 2b.

### Vergleichsbeispiel

Zum Vergleich des Adsorptionsverhaltens des erfindungsgemäßen Adsorbermaterials wurden die zur Zeit in der Medizin verbreitete Aktivkohle nach dem oben beschriebenen Verfahren konditioniert und sowohl dem statischen als auch dem dynamischen Bilirubintest unterworfen. Die Fig. 2 zeigt das Verhalten der Aktivkohle und der erfindungsgemäßen Adsorbermaterialien im statischen und dynamischen Test nebeneinander.

Die Adsorptionsgeschwindigkeit des erfindungsgemäßen Adsorbermaterials ist wesentlich höher als die der zur Zeit eingesetzten Aktivkohle (statischer Test, Fig. 2a). Die Aktivkohle adsorbiert innerhalb 1 h ca. 60 % des Bilirubins aus der B-HSA-Lösung. Im Vergleich dazu adsorbiert das Adsorbermaterial unter den gleichen Bedingungen fast das gesamte Bilirubin (98-100%).

Im dynamischen Test zeigt sich die Überlegenheit des Adsorbermaterials noch deutlicher (Fig. 2b). Während die Aktivkohle unter dynamischen Bedingungen so gut wie nicht adsorbieren kann, zeigt das erfindungsgemäße Adsorbermaterial eine exponentiell verlaufende Abnahme der Bilirubinkonzenträtion. Nach 7 Durchgängen durch das Adsorberbett beträgt die relative Bilirubin-Restkonzentration im Filtrat ca. 25 %. Dieses Ergebnis läßt eine deutliche Verkürzung der Behandlungszeit, eine Steigerung der Wirksamkeit und eine Erhöhung der Überlebenschancen von Patienten mit akutem Leberversagen erwarten.

### Beispiel 3

200 mg des Adsorbermaterials AM1 wurden in eine 6-ml-SPE-Säule eingewogen und mit 5 ml 70 Ma%iger Ethanollösung, 5 ml destilliertem Wasser und 5 ml 0,9 %iger NaCl-Lösung konditioniert. Das so vorbereitete Adsorbermaterial wurde anschließend mit 2 ml einer Gallensäurelösung (c = 1 mg/ml) in 0,9 %iger NaCl-Lösung beladen. 20 µl des Eluats wurden dann auf eine GPC-Säule HEMA 2000 gegeben, mit 0,9 %iger NaCl eluiert und mit RI-Detektor detektiert. Die Beladung mit Gallensäurelösung wurde so oft wiederholt, bis im Chromatogramm ein Peak der Gallensäuren nachgewiesen werden konnte. Aus der Anzahl der Beladungsschritte bis zu diesem Zeitpunkt wurde die Kapazität des Adsorbermaterials zu 210 mg Gallensäuren pro Gramm Adsorbermaterial AM1 ermittelt.

## Patentansprüche

1. Adsorbermaterial auf der Basis vernetzter, poröser Imidazol-Divinylbenzen-Copolymere, hergestellt durch radikalische Suspensionspolymerisation eines Monomergemisches aus Divinylbenzen-Vernetzer und einem Imidazol-Derivat **gekennzeichnet dadurch, dass**
- die Polymerisation in Gegenwart von Luft und/oder Sauerstoff, eines Salzes, eines Stabilisators und eines Inertstoffes erfolgt;
- der Divinylbenzen-Vernetzer mit mindestens 50% Gew. % im Adsorbermaterial enthalten ist;
- das Imidazol-Derivat mit einem Anteil von 4 Gew. % bis 30 Gew. % im Adsorbermaterial enthalten ist;
- es eine spezifische Oberfläche von 200 m²/g bis 900 m²/g und ein Gesamtporenvolumen von 1,0 cm³/g bis 2,0 cm³/g und einen mittleren Porendurchmesser von 100 Å bis 500 Å aufweist, wobei in 1 g des Materials bis 0,3 cm³ Mikroporen, bis 1,2 cm³ Mesoporen und bis 0,5 cm³ Makroporen enthalten sind,
- es vorwiegend kugelförmig in einem Korngrößenbereich von 1 µm bis 300 µm, vorzugsweise 50 µm bis 200 µm oder 1µm bis 50 µm ausgebildet ist.

2. Adsorbermaterial nach Anspruch 1, **gekennzeichnet dadurch, dass** das polymerisierbare Imidazol-Derivat 1-Vinylimidazol, 4-Vinylimidazol, 1-Vinyl-2-methylimidazol, 1-Vinyl-2-ethylimidazol. 1-Propenyl-2-imidazol und 1-Allyl-2-methylimidazol allein oder ein Gemisch davon ist.

3. Adsorbermaterial nach Anspruch 1, **gekennzeichnet dadurch, dass** das Divinylbenzen-Copolymere aus 50 Gew% bis 85 Gew% einpolymerisierten isomeren Divinylbenzen-Monomeren und 5 Gew% bis 40 Gew% einpolymerisierten isomeren Ethyl-Vinyl-Benzen-Monomeren besteht.

4. Adsorbermaterial nach Anspruch 1, **gekennzeichnet dadurch, dass** der Suspensionsstabilisator Polyvinylalkohol oder Methylcellulose oder Hydroxyethylcellulose oder Calciumphosphat oder Aluminiumhydroxid oder Magnesiumhydroxid ist.

5. Adsorbermaterial nach Anspruch 1, **gekennzeichnet dadurch, dass** der Inertstoff Toluen, Ethylacetat, Butylacetat, Dichlorethan, Tetrachlorkohlenstoff einzeln oder ein Gemisch davon ist.

6. Verwendung des Adsorbermaterials nach den Ansprüchen 1 bis 5 im Molecular Adsorbent Recirculating System.

7. Verfahren der Suspensionspolymerisation zur Herstellung eines Adsorbermaterials nach den Ansprüchen 1 bis 6 bei dem die wäßrige Phase 5 Gew% bis 25 Gew% eines Salzes sowie 0,5 Gew% bis 5 Gew% eines Suspensionsstabilisators enthält, die organische Phase 25 Gew% bis 50 Gew% eines Inertstoffes enthält und die Polymerisation in Gegenwart von Luft und/oder Sauerstoff durchgeführt wird.

8. Verfahren nach Anspruch 7, **gekennzeichnet dadurch, dass** als Inertstoff Toluen, Ethylacetat, Butylacetat, Dichlorethan oder Tetrachlorkohlenstoff oder ein Gemisch davon verwendet wird.

9. Verfahren nach Anspruch 8, **gekennzeichnet dadurch, dass** als Suspensionsstabilisator Polyvinylalkohol oder Methylcellulose oder Hydroxyethylcellulose oder Calciumphosphat oder Aluminiumhydroxid oder Magnesiumhydroxid verwendet wird.

## Claims

1. Adsorbent material, based on cross-linked, porous imidazole-divinylbenzene copolymers, formed by radical suspension polymerization of a monomer mixture of divinylbenzene cross-linker and an imidazole derivative, **characterized in that**
- the polymerization is conducted in the presence of air and/or oxygen, a salt, a stabilizer, and an inert substance;
- the adsorbent material contains at least 50 wt% of the divinylbenzene cross-linker;
- the adsorbent material contains 4 wt% to 30 wt% of the imidazole derivative;
- the adsorbent material has a specific surface from 200 m²/g to 900 m²/g and a total pore volume from 1.0 cm³/g to 2.0 cm³/g and an average pore diameter from 100 Å to 500 Å, where 1 g of the material contains up to 0.3 cm³ micropores, up to 1.2 cm³ mesopores, and up to 0.5 cm³ macropores;
- the adsorbent material is essentially of spherical shape having a particle size range from 1 µm to 300 µm, preferably from 50 µm to 200 µm or 1 µm to 50 µm;

2. An adsorbent material of claim 1, **characterized in that** the polymerizable imidazole derivative is 1-vinylimidazole, 4-vinylimidazole, 1-vinyl-2-methylimidazole, 1-vinyl-2-ethylimidazole, 1-propenyl-2-imidazole, 1-allyl-2-methylimidazole, exclusively or a mixture thereof.

3. An adsorbent material of claim 1, **characterized in that** the divinylbenzene copolymer comprises 50 wt% to 85 wt% of polymerised isomeric divinylbenzene monomers and 5 wt% to 40 wt% of polymerised isomeric ethylvinylbenzene monomers.

4. An adsorbent material of claim 1, **characterized in that** the suspension stabilizer comprises poly(vinyl alcohol) or methyl cellulose or hydroxyethyl cellulose or calcium phosphate or aluminium hydroxide or magnesium hydroxide.

5. An adsorbent material of claim 1, **characterized in that** the inert substance comprises toluene, ethyl acetate, butyl acetate, dichloroethane, carbon tetrachloride, exclusively or as a mixture.

6. Utilisation of the adsorbent material of claims 1 to 5 in the Molecular Adsorbent Recirculating System.

7. A method of suspension polymerization to produce the adsorbent material of claims 1 to 5, **characterized in that** the aqueous phase comprises 5 wt% to 25 wt% of a salt and 0.5 wt% to 5 wt% of a suspension stabilizer, the organic phase comprises 25 wt% to 50 wt% of an inert substance, and the polymerization is conducted in the presence of air and/or oxygen.

8. The method of claim 7, **characterized in that** the inert substance comprises toluene, ethyl acetate, butyl acetate, dichloroethane, carbon tetrachloride, exclusively or as a mixture.

9. The method of claim 8, **characterized in that** the suspension stabilizer comprises poly(vinyl alcohol) or methyl cellulose or hydroxyethyl cellulose or calcium phosphate or aluminium hydroxide or magnesium hydroxide.

## Revendications

1. Matériau adsorbant à base de copolymères d'imidazole-divinylbenzène réticulés poreux, fabriqués par polymérisation radicalaire en suspension d'un mélange monomère composé d'un réticulant de divinylbenzène et d'un dérivé d'imidazole, **caractérisé en ce que**
- la polymérisation a lieu en présence d'air et/ou d'oxygène, d'un sel, d'un stabilisant et d'un inerte ;
- le matériau adsorbant comprend le réticulant de divinylbenzène au moins à 50% en poids ;
- le matériau adsorbant comprend une part de dérivé d'imidazole comprise entre 4% et 30% en poids ;
- il présente une surface spécifique comprise entre 200 m²/g et 900 m²/g et un volume de pores total compris entre 1,0 cm³/g et 2,0 cm³/g et un diamètre de pores moyen compris entre 100 Å et 500 Å, 1 g de matériau comprenant jusqu'à 0,3 cm³ de micropores, jusqu'à 1,2 cm³ de mésopores et jusqu'à 0,5 cm³ de macropores ;
- il se présente principalement sous forme de sphères d'un calibre compris entre 1 µm et 300 µm, de préférence entre 50 µm et 200 µm ou entre 1 µm et 50 µm.

2. Matériau adsorbant selon la revendication 1, **caractérisé en ce que** le dérivé d'imidazole polymérisable est du 1-vinylimidazole, 4-vinylimidazole, 1-vinyle-2-méthylimidazole, 1-vinyle-2-éthylimidazole, 1-propényle-2-imidazole et 1-allyle-2-méthylimidazole seul ou en mélange.

3. Matériau adsorbant selon la revendication 1, **caractérisé en ce que** le copolymère de divinylbenzène se compose de 50% à 85% en poids de monomères de divinylbenzène isomères polymérisés et de 5% à 40% en poids de monomères d'éthyle-vinyle-benzène isomères polymérisés.

4. Matériau adsorbant selon la revendication 1, **caractérisé en ce que** le stabilisant de suspension est de l'alcool de polyvinyle ou de la méthylcellulose ou de l'hydroxyéthylcellulose ou du phosphate de calcium ou de l'hydroxyde d'aluminium ou de l'hydroxyde de magnésium.

5. Matériau adsorbant selon la revendication 1, **caractérisé en ce que** l'inerte est du toluène, de l'acétate éthylique, de l'acétate butylique, du dichloréthane, du carbone tétrachloride seul ou en mélange.

6. Utilisation d'un matériau adsorbant selon les revendications 1 à 5 dans le système de recyclage à adsorbant moléculaire (Molecular Adsorbent Recirculating System).

7. Procédé de polymérisation en suspension pour la fabrication d'un matériau adsorbant selon les revendications 1 à 6, pour lequel la phase aqueuse comprend de 5% à 25% en poids de sel ainsi que de 0,5% à 5% en poids de stabilisant de suspension, la phase organique comprend de 25% à 50% en poids d'inerte et la polymérisation est réalisée en présence d'air et/ou d'oxygène.

8. Procédé selon la revendication 7, **caractérisé en ce que** du toluène, de l'acétate éthylique, de l'acétate butylique, du dichloréthane ou du carbone tétrachloride ou un mélange de ceux-ci sont utilisés comme inerte.

9. Procédé selon la revendication 8, **caractérisé en ce que** de l'alcool de polyvinyle ou de la méthylcellulose ou de l'hydroxyéthylcellulose ou du phosphate de calcium ou de l'hydroxyde d'aluminium ou de l'hydroxyde de magnésium sont utilisés comme stabilisant de suspension.
